# EUROPEAN PATENT APPLICATION

(11) **EP 3 712 257 A1**
(43) Date of publication of application: **23.09.2020**
(21) Application number: 19164241.2
(22) Date of filing: 21.03.2019
(51) Int. Cl.: C12N 5/0783, C12N 5/10, A61K 35/17, A61P 35/02

(54) **MODIFIED NATURAL KILLER CELLS WITH INCREASED RESISTANCE TO CELL DEATH**

(71) Applicant: ONK Therapeutics Limited, Co. Galway H91 V6KV (IE)
(72) Inventor: The designation of the inventor has not yet been filed
(74) Representative: Schlich, George

(57) **Abstract**

NK cells and NK cell lines are modified to have increased resistance to TRAIL-induced cell death, such as by knockout of a TRAIL receptor or by linking a TRAIL receptor to a NK cell co-stimulatory domain.

SEQUENCE LISTING

<110>  Onkimmune Limited

<120>  MODIFIED NATURAL KILLER CELLS WITH INCREASED RESISTANCE TO CELL
       DEATH

<130>  P40540EP

<160>  3

<170>  PatentIn version 3.5

<210>  1
<211>  19
<212>  RNA
<213>  Homo sapiens

<400>  1
cccaucuuga acauaccag                                                    19


<210>  2
<211>  20
<212>  RNA
<213>  Homo sapiens

<400>  2
   aaccggugca cagagggugu                                                    20


<210>  3
<211>  20
<212>  RNA
<213>  Homo sapiens

<400>  3
auuuacaagc uguacauggg                                                   20

## Description

### Introduction

The present invention relates to the modification of natural killer (NK) cells and NK cell lines to produce useful derivatives thereof. Furthermore, the present invention relates to methods of producing modified NK cells and NK cell lines, compositions containing the cells and cell lines and uses of said cells and compositions comprising the cells in the treatment of cancer.

### Background to the Invention

Typically, immune cells require a target cell to present antigen via major histocompatibility complex (MHC) before triggering an immune response resulting in the death of the target cell. This allows cancer cells not presenting MHC class I to evade the majority of immune responses.

NK cells are able, however, to recognize cancer cells in the absence of MHC class I expression. Hence they perform a critical role in the body's defence against cancer.

On the other hand, in certain circumstances, cancer cells demonstrate an ability to dampen the cytotoxic activity of NK cells, through expression of ligands that bind inhibitory receptors on the NK cell membrane. Resistance to cancer can involve a balance between these and other factors.

Cytotoxicity, in this context, refers to the ability of immune effector cells, e.g. NK cells, to induce cancer cell death, e.g. by releasing cytolytic proteins, or by binding receptors on cancer cell membranes and inducing apoptosis of said cancer cells. Cytotoxicity is affected not only by signals that induce release of cytolytic proteins but also by signals that inhibit their release. An increase in cytotoxicity will therefore lead to more efficient killing of cancer cells, with less chance of the cancer cell dampening the cytotoxic activity of the NK, as mentioned above.

Genetic modification to remove inhibitory receptor function on NK cells has been suggested as a method for increasing the cytotoxicity of NK cells against cancer cells that lack MHC class I expression but are able to dampen NK cytotoxicity (Bodduluru *et al.* 2012). NKG2A has been established as an inhibitory receptor worth silencing under these circumstances, as certain cancer cells are known to express HLA-E which binds NKG2A and inhibits NK cell cytotoxicity in the absence of MHC class I expression (Shook et al. 2011; WO 2006/023148) (Campana D. *et al*. 2019).

Another method of downregulating NKG2A expression has been shown in NK-92 cells, in which transfection with a gene encoding IL-15 was shown to be associated with a reduction in NKG2A expression (Zhang et al. 2004). However, despite an observed increase in the cytotoxicity of the NK cells, the increase was likely a result of a concomitant increase in expression of the activating receptor NKG2D. This is supported by the observation that blocking NKG2A receptors on NK-92 cells was not associated with an increase in cytotoxicity against multiple myeloma cells (Heidenreich et al. 2012). Nevertheless, it is worth noting that the NK-92 cell line is a highly cytotoxic cell line with very low expression of inhibitory receptors. Therefore, any increase in cytotoxicity associated with decreased NKG2A expression might have been too trivial to detect.

Similar studies have been carried out in mice. For example, mice express a receptor called Ly49 on NK cells, which is analogous to human inhibitory KIR receptors. It has been shown that by blocking the Ly49 receptor with antibody fragments, NK cells are more cytotoxic and capable of killing murine leukemia cells *in vitro* and *in vivo* (Koh et al. 2001).

It is a consequence of reducing inhibitory receptor function, however, that 'normal' cells in the body also become more susceptible to attack by modified NK cells, as the modified NK cells become less capable of distinguishing between 'normal' cells and cancer cells. This side effect is a significant disadvantage of reducing 'classical' inhibitory receptor function.

Another way in which NK cells are known to kill cancer cells is by expressing TRAIL on their surface. TRAIL ligand is able to bind TRAIL receptors on cancer cells and induce apoptosis of said cancer cells. One speculative approach describes overexpressing membrane bound TRAIL ligand on NK cells, in order to take advantage of this anti-cancer mechanism (EP1621550). Furthermore, IL-12 has been reported to upregulate TRAIL expression on NK cells (Smyth et al. 2001). Nevertheless, cancer cells have developed evasive and protective mechanisms for dealing with NK cells expressing TRAIL. Decoy TRAIL receptors are often expressed on cancer cell membranes, and binding of TRAIL to these decoy receptors (e.g. DcR1 and DcR2) is unable to induce apoptosis; methods of overcoming such mechanisms have not yet been pursued.

Another problem with using NK cells expressing TRAIL to target cancer cells is that certain NK cells express TRAIL receptors themselves. TRAIL expression on NK cells can thus, in some cases, lead to fratricide.

Acute myeloid leukemia (AML) is a hematopoietic malignancy involving precursor cells committed to myeloid development, and accounts for a significant proportion of acute leukemias in both adults (90%) and children (15-20%) (Hurwitz, Mounce et al. 1995; Lowenberg, Downing et al. 1999). Despite 80% of patients achieving remission with standard chemotherapy (Hurwitz, Mounce et al. 1995; Ribeiro, Razzouk et al. 2005), survival remains unsatisfactory because of high relapse rates from minimal residual disease (MRD). The five-year survival is age-dependent; 60% in children (Rubnitz 2012), 40% in adults under 65 (Lowenberg, Downing et al. 1999) and 10% in adults over 65 (Ferrara and Schiffer 2013). These outcomes can be improved if patients have a suitable hematopoietic cell donor, but many do not, highlighting the need for an alternative approach to treatment.

Natural killer (NK) cells are cytotoxic lymphocytes, with distinct phenotypes and effector functions that differ from e.g. natural killer T (NK-T) cells. For example, while NK-T cells express both CD3 and T cell antigen receptors (TCRs), NK cells do not. NK cells are generally found to express the markers CD16 and CD56, wherein CD16 functions as an Fc receptor and mediates antibody dependent cell-mediated cytotoxicity (ADCC) which is discussed below. KHYG-1 is a notable exception in this regard. Despite NK cells being naturally cytotoxic, NK cell lines with increased cytotoxicity have been developed. NK-92 and KHYG-1 represent two NK cell lines that have been researched extensively and show promise in cancer therapeutics (Swift et al. 2011; Swift et al. 2012).

Adoptive cellular immunotherapy for use in cancer treatment commonly involves administration of natural and modified T cells to a patient. T cells can be modified in various ways, e.g. genetically, so as to express receptors and/or ligands that bind specifically to certain target cancer cells. Transfection of T cells with high-affinity T cell receptors (TCRs) and chimeric antigen receptors (CARs), specific for cancer cell antigens, can give rise to highly reactive cancer-specific T cell responses. A major limitation of this immunotherapeutic approach is that T cells must either be obtained from the patient for autologous *ex vivo* expansion or MHC-matched T cells must be used to avoid immunological eradication immediately following transfer of the cells to the patient or, in some cases, the onset of graft-vs-host disease (GVHD). Additionally, successfully transferred T cells often survive for prolonged periods of time in the circulation, making it difficult to control persistent side-effects resulting from treatment.

In the setting of haploidentical bone marrow transplantation, the graft-versus-leukemia effect is believed to be mediated by NK cells when there is a KIR inhibitory receptor-ligand mismatch, which can lead to improved survival in the treatment of AML (Ruggeri, Capanni et al. 2002; Ruggeri, Mancusi et al. 2005). Furthermore, rapid NK recovery is associated with better outcome and a stronger graft-vs-leukemia (GVL) effect in patients undergoing T-depleted hematopoietic cell transplantation (HCT) in AML (Savani, Mielke et al. 2007). Other trials have used haploidentical NK cells expanded *ex vivo* to treat AML in adults (Miller, Soignier et al. 2005) and children (Rubnitz, Inaba et al. 2010).

Several permanent NK cell lines have been established, and the most notable is NK-92, derived from a patient with non-Hodgkin's lymphoma expressing typical NK cell markers, with the exception of CD16 (Fc gamma receptor III). NK-92 has undergone extensive preclinical testing and exhibits superior lysis against a broad range of tumours compared with activated NK cells and lymphokine-activated killer (LAK) cells (Gong, Maki et al. 1994). Cytotoxicity of NK-92 cells against primary AML has been established (Yan, Steinherz et al. 1998).

Another NK cell line, KHYG-1, has been identified as a potential contender for clinical use (Suck et al. 2005) but has reduced cytotoxicity so has received less attention than NK-92. KHYG-1 cells are known to be pre-activated. Unlike endogenous NK cells, KHYG-1 cells are polarized at all times, increasing their cytotoxicity and making them quicker to respond to external stimuli. NK-92 cells have a higher baseline cytotoxicity than KHYG-1 cells.

It is therefore clear that current adoptive immunotherapy protocols are affected by donor variability in the quantity and quality of effector cells, variables that could be eliminated if effective cell lines were available to provide more standardized therapy.

A considerable amount of research into NK cell cytotoxicity has been performed using mouse models. One example is the finding that perforin and granzyme B mRNA are constitutively transcribed in mouse NK cells, but minimal levels of protein are detected until stimulation or activation of the NK cells (Fehniger et al, 2007). Although this work and other work using mouse NK cells is of interest, it cannot be relied upon as conclusive evidence for NK cell cytotoxicity in humans. In contrast to the above example, human NK cells express high levels of perforin and granzyme B protein prior to stimulation (Leong et al, 2011). The result being that when either mouse or human NK cells are freshly isolated in culture, the mouse NK cells have weak cytolytic activity, whereas the human NK cells exhibit strong cytolytic capabilities.

Mouse and human NK cells also vary greatly in their expression markers, signalling cascades and tissue distribution. For example, CD56 is used as a marker for human NK cells, whereas mouse NK cells do not express this marker at all. Furthermore, a well-established mechanism for regulating NK cell cytotoxicity is via ligand binding NK activation and inhibitory receptors. Two of the most prominent human NK activation receptors are known to be NKp30 and NKp44, neither of which are expressed on mouse NK cells. With regards to NK inhibitory receptors, whilst human NK cells express KIRs that recognise MHC class I and dampen cytotoxic activity, mouse NK cells do not express KIRs at all but, instead, express Ly49s (Trowsdale et al, 2001). All in all, despite mouse NK cells achieving the same function as human NK cells in their natural physiological environment, the mechanisms that fulfil this role vary significantly between species.

Thus there exists a need for alternative and preferably improved human NK cells and human NK cell lines, e.g. with a more cytotoxic profile.

An object of the invention is to provide NK cells and NK cell lines that are less vulnerable to TRAIL-induced cell death and/or have a more cytotoxic phenotype. A further object is to provide methods for producing modified NK cells and NK cell lines, compositions containing the cells or cell lines and uses of said compositions in the treatment of cancers. More particular embodiments aim to provide treatments for identified cancers, e.g. blood cancers, such as leukemias, as well as solid tumors. Specific embodiments aim at combining two or more modifications of NK cells and NK cell lines to further enhance the cytotoxicity of the modified cells.

### Summary of the Invention

There are provided herein modified NK cells and NK cell lines that are resistant to TRAIL-induced cell death and, optionally, have a more cytotoxic phenotype. Further still, methods of making the cells and cell lines are provided. Also provided are compositions of modified NK cells and NK cell lines, and uses of said cells and compositions for treating cancer.

The invention provides methods of modifying NK cells and NK cell lines using, for example, genetic engineering to knock out and/or knock down genes encoding TRAIL receptors, inhibitory receptors and/or checkpoint inhibitory receptors, express genes encoding modified TRAIL receptors, TRAIL ligands and variants, and/or express genes encoding chimeric antigen receptors (CARs) and/or Fc receptors.

Furthermore, compositions of the invention include NK cells and NK cell lines in which two or more modifications are provided, wherein multiple modifications further enhance the cytotoxic activity of the cells and cell lines.

The invention provides NK cells or NK cell lines that have been modified to have increased resistance to TRAIL-induced cell death. Preferably, the NK cells or NK cell lines have been modified (e.g. genetically) to have reduced function of one or more TRAIL receptors.

According to the invention, there are further provided methods of treating cancer, e.g. blood cancers and solid tumours, using modified NK cell lines, e.g. NK-92 cells and derivatives thereof, wherein the modified NK cell lines are optionally further engineered to lack expression of checkpoint inhibitory receptors, express TRAIL ligand variants and/or express CARs and/or Fc receptors.

Diseases particularly treatable according to the invention include cancers, blood cancers, leukemias and specifically acute myeloid leukemia. Tumours and cancers in humans in particular can be treated. References to tumours herein include references to neoplasms.

### Details of the Invention

Accordingly, the present invention provides a natural killer (NK) cell or NK cell line that has been modified to resist TRAIL-induced cell death. The NK cells may be less vulnerable to TRAIL-induced cell death or fratricide as a result.

As described in detail below, NK cells and NK cell lines have been genetically modified so as to increase their resistance to TRAIL-induced cell death and, optionally, increase their cytotoxic activity against cancer cells.

Together, the NK cells and NK cell lines of the invention will be referred to as the NK cells (unless the context requires otherwise).

In a preferred series of embodiments, the NK cells have been modified to have reduced function of one or more TRAIL receptors. This is optionally achieved using gene knockout or knockdown (e.g. using siRNA) or restricting the expression of the TRAIL receptor within the cell endoplasmic reticulum. Advantageously, the modified cells are more resistant to cell death by way of TRAIL signaling.

Preferably, DR4 and/or DR5 function is reduced on the NK cells of the invention. It is particularly preferred that the DR4 and/or DR5 genes are knocked out. If multiple copies of the genes are present it is preferred that all are knocked out.

The NK cells may be modified in a way that both reduces TRAIL-induced death of the NK cells and provides the NK cells with a more cytotoxic phenotype. Preferably the same modification can achieve both of these advantages.

The resistance of NK cells according to the invention against TRAIL-induced cell death is preferably increased by at least 10%, more preferably at least 20%, more preferably at least 50%, most preferably at least 100%, relative to wildtype NK cells.

It is preferred that the NK cells are modified to express a TRAIL receptor linked to a co-stimulatory domain. Preferably, the TRAIL receptor is selected from DR4 and DR5. Preferably, the co-stimulatory domain is selected from 4-1BB, CD28, DAP-10, DAP-12, CD278 (ICOS) and OX40. More preferably, the co-stimulatory domain is 4-1BB linked to CD3zeta.

Co-stimulatory domains are protein domains that provide signals in addition to those sent by the antigen receptor (e.g. DR4 or DR5) and are able to help induce full activation of a lymphocyte (e.g. NK cell). Full activation of NK cells results in cytokine production, sustained proliferation, anti-apoptotic intracellular signaling and enhanced sensitivity to further stimulation through the antigen receptor. Overall, this provides the NK cells with a more cytotoxic phenotype. Binding of ligand to receptor linked to a costimulatory domain thus activates this cell stimulatory consequence of ligand binding.

Increased or enhanced cytotoxicity resulting from modification of an NK cell is defined by comparison to the cytotoxicity of a wildtype cell not having such modification. A wildtype cell is defined as a cell of the same type as that comprising the modification but not having the modification itself. For example, the invention provides NK-92 cells modified to express DR5 linked to co-stimulatory domain CD28; these NK cells are modified to exhibit increased cytotoxicity against cancer cells, relative to wildtype NK-92 cells (i.e. NK-92 cells not modified to express DR5 linked to CD28).

KHYG-1 cells typically express low levels of DR4 and DR5 when resting. Nevertheless, expression of DR4 and/or DR5 may be upregulated when the cells are activated or under the influence of / exposed to exogenous factors. As such, KHYG-1 cells are also suitably modified according to the invention.

The cytotoxicity of NK cells according to the invention against target (e.g. cancer) cells is preferably increased by at least 10%, more preferably at least 20%, more preferably at least 50%, most preferably at least 100%, relative to wildtype NK cells. Cytotoxicity is easily measured using e.g. known methods for determining target cell death during co-incubation with the NK cells.

Advantageously, TRAIL receptors linked to one or more co-stimulatory domains compete with existing TRAIL receptors on the surface of the NK cells of the invention and thus limit binding of TRAIL to these death-inducing TRAIL receptors. Further still, NK cells expressing a TRAIL receptor linked to a co-stimulatory domain produce activating signals upon binding of TRAIL which increase the cytotoxic activity of the NK cells. This can be a particularly advantageous feature when using the modified NK cells to treat cancer.

In all series of embodiments of the invention NK cells may be provided having reduced or absent checkpoint inhibitory receptor function. Such NK cells of the invention preferably have one or more checkpoint inhibitory receptor genes knocked out. Preferably, these receptors are specific checkpoint inhibitory receptors. Preferably still, these checkpoint inhibitory receptors are one or more or all of CD96 (TACTILE), CD152 (CTLA4), CD223 (LAG-3), CD279 (PD-1), CD328 (SIGLEC7), SIGLEC9, TIGIT and TIM-3.

In other embodiments, NK cells are provided in which one or more inhibitory receptor signaling pathways are knocked out or exhibit reduced function - the result again being reduced or absent inhibitory receptor function. For example, signaling pathways mediated by SHP-1, SHP-2 and/or SHIP are knocked out by genetic modification of the cells.

The resulting NK cells exhibit improved cytotoxicity and are of greater use therefore in cancer therapy, especially blood cancer therapy, and in particular in treatment of leukemias and/or multiple myeloma.

The genetic modification may occur before the cell has differentiated into an NK cell. For example, pluripotent stem cells (e.g. iPSCs) can be genetically modified to lose the capacity to express one or more checkpoint inhibitory receptors. The modified iPSCs are then differentiated to produce genetically modified NK cells with increased cytotoxicity.

It is preferred to reduce function of checkpoint inhibitory receptors over other inhibitory receptors, due to the expression of the former following NK cell activation. The normal or 'classical' inhibitory receptors, such as the majority of the KIR family, NKG2A and LIR-2, bind MHC class I and are therefore primarily involved in reducing the problem of self-targeting. Preferably, therefore, checkpoint inhibitory receptors are knocked out. Reduced or absent function of these receptors according to the invention prevents cancer cells from suppressing immune effector function (which might otherwise occur if the receptors were fully functional). Thus a key advantage of these embodiments of the invention lies in NK cells that are less susceptible to suppression of their cytotoxic activities by cancer cells; as a result they are useful in cancer treatment.

As used herein, references to inhibitory receptors generally refer to a receptor expressed on the plasma membrane of an immune effector cell, e.g. a NK cell, whereupon binding its complementary ligand results in intracellular signals responsible for reducing the cytotoxicity of said immune effector cell. These inhibitory receptors are expressed during both 'resting' and 'activated' states of the immune effector cell and are often associated with providing the immune system with a 'self-tolerance' mechanism that inhibits cytotoxic responses against cells and tissues of the body. An example is the inhibitory receptor family 'KIR' which are expressed on NK cells and recognize MHC class I expressed on healthy cells of the body.

Also as used herein, checkpoint inhibitory receptors are usually regarded as a subset of the inhibitory receptors referred to generally above. Unlike other inhibitory receptors, however, checkpoint inhibitory receptors are expressed at higher levels during prolonged activation and cytotoxicity of an immune effector cell, e.g. a NK cell. This phenomenon is useful for dampening chronic cytotoxicity at, for example, sites of inflammation. Examples include the checkpoint inhibitory receptors PD-1, CTLA-4 and CD96, all of which are expressed on NK cells.

NK cells of the invention may also lack a gene encoding a checkpoint inhibitory receptor selected from CD96 (TACTILE), CD152 (CTLA4), CD223 (LAG-3), CD279 (PD-1), CD328 (SIGLEC7), SIGLEC9, TIGIT and TIM-3.

A NK cell lacking a gene can refer to either a full or partial deletion, mutation or otherwise that results in substantially no functional gene product being expressed. The inventors have previously shown the cytotoxic effects of using siRNA to knock down expression of checkpoint inhibitory receptors in KHYG-1 cells. CD96 knockdown (KD) KHYG-1 cells demonstrated enhanced cytotoxicity against leukemia cells at a variety of effector:target (E:T) ratios. In embodiments, the NK cell lacks genes encoding two or more of the inhibitory receptors.

More specific embodiments comprise a NK cell lacking a gene encoding a checkpoint inhibitory receptor selected from CD96 (TACTILE), CD152 (CTLA4) and CD279 (PD-1).

In further preferred embodiments, the NK cell is a derivative of a primary NK cell or a NK-92 NK cell. The NK cell may also be a derivative of a KHYG-1 cell.

It is preferred that the NK cells of the invention further express a mutant (variant) TRAIL ligand. Cytotoxicity-enhancing modifications of the NK cells hence also include increased expression of both TRAIL ligand and/or mutated TRAIL ligand variants.

The resulting NK cells exhibit increased binding to TRAIL receptors and, as a result, increased cytotoxicity against cancers, especially blood cancers, in particular leukemias. However, NK cells of the invention are less susceptible to TRAIL-induced cell death due to the modifications described above and below.

The mutants / variants preferably have lower affinity (or in effect no affinity) for 'decoy' receptors, compared with the binding of wild type TRAIL to decoy receptors. Such decoy receptors represent a class of TRAIL receptors that bind TRAIL ligand but do not have the capacity to initiate cell death and, in some cases, act to antagonize the death signaling pathway. Mutant / variant TRAIL ligands may be prepared according to WO 2009/077857.

The mutants / variants may separately have increased affinity for TRAIL receptors, e.g. DR4 and DR5. Wildtype TRAIL is typically known to have a K_{D} of >2 nM for DR4, >5 nM for DR5 and >20 nM for the decoy receptor DcR1 (WO 2009/077857; measured by surface plasmon resonance), or around 50 to 100 nM for DR4, 1 to 10 nM for DR5 and 175 to 225 nM for DcR1 (Truneh, A. et al. 2000; measured by isothermal titration calorimetry and ELISA). Therefore, an increased affinity for DR4 is suitably defined as a K_{D} of <2 nM or <50 nM, respectively, whereas an increased affinity for DR5 is suitably defined as a K_{D} of <5 nM or <1 nM, respectively as per the assays defined. A reduced affinity for decoy receptor DcR1 is suitably defined as a K_{D} of >50 nM or >225 nM, respectively. In any case, an increase or decrease in affinity exhibited by the TRAIL variant/mutant is relative to a baseline affinity exhibited by wildtype TRAIL. The affinity is preferably increased at least 10%, at least 25%, at least 50%, more preferably at least 100% compared with that exhibited by wildtype TRAIL.

The TRAIL variant preferably has an increased affinity for one or both of DR4 and DR5 as compared with its affinity for wildtype TRAIL. Preferably, the affinity is at least 1.5-fold, 2-fold, 5-fold, 10-fold, 100-fold, or even 1,000-fold or greater for DR4 and/or DR5 than for wildtype TRAIL.

The TRAIL variant may in particular cases have an increased affinity for DR5 as compared with its affinity for DR4, DcR1 and DcR2. Preferably, the affinity is at least 1.5-fold, 2-fold, 5-fold, 10-fold, 100-fold, or even 1,000-fold or greater for DR5 than for one or more of DR4, DcR1 and DcR2. More preferably, the affinity is at least 1.5-fold, 2-fold, 5-fold, 10-fold, 100-fold, or even 1,000-fold or greater for DR5 than for at least two, and preferably all, of DR4, DcR1 and DcR2.

Further specific embodiments comprise a NK cell expressing a mutant TRAIL ligand that has reduced or no affinity for TRAIL decoy receptors. Preferably, this NK cell is a derivative of NK-92. Further specific embodiments comprise a NK cell expressing a mutant TRAIL ligand that has reduced or no affinity for TRAIL decoy receptors and increased affinity for DR4 and/or DR5.

A key advantage of these embodiments of the invention lies in NK cells that have multiple modifications compared to wildtype cells and greater potency in killing cancer cells.

In certain embodiments, the TRAIL variant comprises at least one amino acid substitution at a position selected from the group consisting of 131, 149, 159, 160, 189, 191, 193, 195, 199, 200, 201, 203, 204, 212, 213, 214, 215, 218, 240, 251, 261, 264, 266, 267, 269, and 270.

In certain embodiments, the TRAIL variant comprises at least one substitution selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, and H270D.

In certain embodiments, the TRAIL variant comprises at least two substitutions selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, and H270D.

In certain embodiments, the TRAIL variant comprises at least three substitutions selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, and H270D.

In certain embodiments, amino acid substitution of the TRAIL variant is selected from the group consisting of G131R, G131K, R149I, R149M, R149N, R149K, S159R, G160E, Y189A, Y189Q, R191K, Q193H, Q193K, Q193S, Q193R, E195R, N199V, N199R, N199H, T200H, K201R, K201H, D203A, K204E, K204D, K204Y, K212R, Y213W, T214R, S215D, S215E, S215H, S215K, S215D, D218H, D218A, Y240A, K251D, K251E, K251Q, T261L, H264R, I266L, D267Q, D269A, D269H, H270D, T214R / E195R, T214R / D269H, Y189A / Q193S / N199V / K201R / Y213W / S215D, Y213W / S215D, N199R / K201H, N199H / K201R, G131R / N199R / K201H, G131R / N199R / K201H / R149I / S159R / S215D, G131R / R149I / S159R/S215D, G131R / N199R / K201H / R149I / S159R / S215D, G131R / D218H, Y189Q / R191K / Q193R / H264R / I266L / D267Q, T261L / G160E, T261L / H270D, T261L / G160E / H270D, and T261L / G160E / H270D / T200H (use of "/" indicates multiple amino acid substitutions).

In certain embodiments, amino acid substitution of the TRAIL variant is selected based on the variant having an increased affinity for DR5; a substitution of this kind may be selected from the group consisting of D269H, E195R, T214R, D269H / E195R, T214R / E195R, T214R / D269H, N199V, Y189A / Q193S / N199V / K201R / Y213W / S215D, Y213W / S215D, D269A and Y240A.

In certain embodiments, amino acid substitution of the TRAIL variant is selected based on the variant having an increased affinity for DR4; a substitution of this kind may be selected from the group consisting of G131R, G131K, R149I, R149M, R149N, R149K, S159R, Q193H, W193K, N199R, N199R / K201H, N199H / K201R, G131R / N199R / K201H, G131R / N199R / K201H, G131R / N199R / K201H / R149I / S159R / S215D, G131R / R149I / S159R / S215D, G131R / D218H, K201R, K201H, K204E, K204D, K204L, K204Y, K212R, S215E, S215H, S215K, S215D, D218H, K251D, K251E, K251Q and Y189Q / R191K / Q193R / H264R / I266L / D267Q.

In certain embodiments, amino acid substitution of the TRAIL variant is selected based on the variant having a decreased affinity for TRAIL decoy receptors; a substitution of this kind may be selected from the group consisting of T261L, H270D, T200H, T261L / G160E, T261L / H270D, T261L / G160E / H270D, T261L / G160E / H270D / T200H, D203A and D218A.

The inventors have previously genetically modified NK cells to express a mutant TRAIL. Modified KHYG-1 and NK-92 cells expressed a mutant TRAIL. The modified KHYG-1 cells exhibited improved cytotoxicity against cancer cell lines *in vitro.* These cell lines express TRAIL receptors (e.g. DR4 and DR5). Other preferred embodiments of the modified NK cells express no or substantially no TRAIL receptors, or do so only at a low level - sufficiently low that viability of the modified NK cells is not adversely affected by expression of the mutant TRAIL. Further still, NK cells with TRAIL receptors linked to co-stimulatory domains can be used as described herein.

In an optional embodiment, treatment of a cancer using modified NK cells expressing TRAIL or a TRAIL variant is enhanced by administering to a patient an agent capable of upregulating expression of TRAIL death receptors on cancer cells. This agent may be administered prior to, in combination with or subsequently to administration of the modified NK cells. It is preferable, however, that the agent is administered prior to administering the modified NK cells. In a preferred embodiment the agent upregulates expression of DR5 on cancer cells. The agent may optionally be a chemotherapeutic medication, e.g. Bortezomib, and administered in a low dose capable of upregulating DR5 expression on the cancer. The invention is not limited to any particular agents capable of upregulating DR5 expression, but examples of DR5-inducing agents include Bortezomib, Gefitinib, Piperlongumine, Doxorubicin, Alpha-tocopheryl succinate and HDAC inhibitors.

The mutant / variant TRAIL ligand may also be linked to one or more NK cell costimulatory domains, e.g. 41BB / CD137, CD3zeta / CD247, DAP12 or DAP10. Binding of the variant to its receptor on a target cell thus promotes apoptotic signals within the target cell, as well as stimulating further cytotoxic signals in the NK cell.

According to further preferred embodiments of the invention, modified NK cells are provided that additionally have reduced checkpoint inhibitory receptor function and also express a mutant TRAIL ligand, as described in more detail above in relation to these respective NK cell modifications.

The present invention also provides NK cells and NK cell lines, preferably NK-92 cells and derivatives thereof, modified to express one or more CARs.

Suitably for cancer therapy uses, the CARs specifically bind to one or more ligands on cancer cells, e.g. CS1 (SLAMF7) on myeloma cells. For use in treating specific cancers, e.g. multiple myeloma, the CAR may bind CD38. For example, the CAR may include the binding properties of e.g. variable regions derived from, similar to, or identical with those from the known monoclonal antibody daratumumab. Such NK cells may be used in cancer therapy in combination with an agent that inhibits angiogenesis, e.g. lenalidomide. For use in therapy of cancers, especially leukemias and AML in particular, the CAR may bind to CLL-1.

The CAR-NKs may be bispecific, wherein their affinity is for two distinct ligands / antigens. Bispecific CAR-NKs can be used either for increasing the number of potential binding sites on cancer cells or, alternatively, for localizing cancer cells to other immune effector cells which express ligands specific to the NK-CAR. For use in cancer therapy, a bispecific CAR may bind to a target tumour cell and to an effector cell, e.g. a T cell, NK cell or macrophage. Thus, for example, in the case of multiple myeloma, a bispecific CAR may bind a T cell antigen (e.g. CD3, etc.) and a tumour cell marker (e.g. CD38, etc.). A bispecific CAR may alternatively bind to two separate tumour cell markers, increasing the overall binding affinity of the NK cell for the target tumour cell. This may reduce the risk of cancer cells developing resistance by downregulating one of the target antigens. An example in this case, in multiple myeloma, would be a CAR binding to both CD38 and CS-1/SLAMF7. Another tumour cell marker suitably targeted by the CAR is a "don't eat me" type marker on tumours, exemplified by CD47.

Optional features of the invention include providing further modifications to the NK cells and NK cell lines described above, wherein, for example, a Fc receptor (which can be CD16, CD32 or CD64, including subtypes and derivatives) is expressed on the surface of the cell. In use, these cells can show increased recognition of antibody-coated cancer cells and improve activation of the cytotoxic response.

Further optional features of the invention include adapting the modified NK cells and NK cell lines to better home to specific target regions of the body. NK cells of the invention may be targeted to specific cancer cell locations. In preferred embodiments for treatment of blood cancers, NK effectors of the invention are adapted to home to bone marrow. Specific NK cells are modified by fucosylation and/or sialylation to home to bone marrow. This may be achieved by genetically modifying the NK cells to express the appropriate fucosyltransferase and/or sialyltransferase, respectively. Increased homing of NK effector cells to tumour sites may also be made possible by disruption of the tumour vasculature, e.g. by metronomic chemotherapy, or by using drugs targeting angiogenesis (Melero et al, 2014) to normalize NK cell infiltration via cancer blood vessels.

Yet another optional feature of the invention is to provide modified NK cells and NK cell lines with an increased intrinsic capacity for rapid growth and proliferation in culture. This can be achieved, for example, by transfecting the cells to overexpress growth-inducing cytokines IL-2 and IL-15. Moreover, this optional alteration provides a cost-effective alternative to replenishing the growth medium with cytokines on a continuous basis.

The invention further provides a method of making a modified NK cell or NK cell line, comprising genetically modifying the cell or cell line as described herein so as to make it more resistant to TRAIL-induced cell death and/or increase its cytotoxicity. This genetic modification can be a stable knockout of a gene, e.g. by CRISPR, or a transient knockdown of a gene, e.g. by siRNA.

In a preferred embodiment, a stable genetic modification technique is used, e.g. CRISPR, in order to provide a new NK cell line (as described above) with increased resistance to TRAIL-induced cell death and increased cytotoxicity, e.g. a derivative of NK-92 cells.

In embodiments, the method is further useful for making a NK cell or NK cell line that has been modified so as to reduce inhibitory receptor function. Preferably, these inhibitory receptors are checkpoint inhibitory receptors.

More specific embodiments comprise a method for making a NK cell or NK cell line with reduced inhibitory receptor function, wherein the checkpoint inhibitory receptors are selected from CD96 (TACTILE), CD152 (CTLA4), CD223 (LAG-3), CD279 (PD-1), CD328 (SIGLEC7), SIGLEC9, TIGIT and TIM-3.

In preferred embodiments, the method comprises modifying the NK cells to reduce function of two or more of the inhibitory receptors.

The invention still further provides a method of making a modified NK cell or NK cell line comprising genetically modifying the cell or cell line to express TRAIL ligand or mutant TRAIL (variant) ligand.

In embodiments, the method comprises modifying a NK cell or NK cell line to express mutant TRAIL ligand that has an increased affinity for TRAIL receptors. Preferably, the TRAIL receptors are DR4 and/or DR5. Preferred embodiments provide a method of modifying the NK cells or NK cell lines to express a mutant TRAIL ligand that has a reduced affinity for decoy TRAIL receptors.

In further preferred embodiments, the method comprises modifying a NK cell or NK cell line to remove function of a checkpoint inhibitory receptor and also to express a mutant TRAIL ligand with reduced or no binding affinity for decoy TRAIL receptors.

Further typical embodiments provide a method for making a NK cell or NK cell line, in which function of one or more checkpoint inhibitory receptors has been removed and/or a mutant TRAIL ligand is expressed, which has reduced or no binding affinity for decoy TRAIL receptors, and the cell is further modified to express a CAR or bispecific CAR. The properties of the CAR are optionally as described above.

In embodiments, the method comprises making a NK cell or NK cell line, in which function of one or more checkpoint inhibitory receptors has been removed and/or a mutant TRAIL ligand is expressed, which has reduced or no binding affinity for decoy TRAIL receptors, and the cell is optionally modified to express a CAR or bispecific CAR, and the cell is further modified to express one or more Fc receptors. Suitable Fc receptors are selected from CD16 (FcRIII), CD32 (FcRII) and CD64 (FcRI).

Preferred embodiments of all the above comprise a method of making NK cells and NK cell lines being a derivative of NK-92.

As per the objects of the invention, the modified NK cell, NK cell line or composition thereof with increased cytotoxicity are for use in treating cancer in a patient, especially blood cancer, especially in a human.

In preferred embodiments, the modified NK cell, NK cell line or composition is for use in treating blood cancers including acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, smoldering multiple myeloma (SMM), active myeloma or light chain myeloma.

In even more preferred embodiments, a NK cell line is obtained as a derivative of NK-92 as described in the invention, optionally additionally modified by reducing checkpoint inhibitory receptor function in a NK-92 cell or expressing a mutant TRAIL ligand in a NK-92 cell, or both, for use in treating blood cancer.

Modified NK cells, NK cell lines and compositions thereof described herein, above and below, are suitable for treatment of cancer, in particular cancer in humans, e.g. for treatment of cancers of blood cells or solid cancers. The NK cells and derivatives are preferably human NK cells. For human therapy, human NK cells are preferably used.

Various routes of administration will be known to the skilled person to deliver active agents and combinations thereof to a patient in need. Embodiments of the invention are for blood cancer treatment. Administration of the modified NK cells and/or NK cell lines can be systemic or localized, such as for example via the intraperitoneal route.

In other embodiments, active agent is administered more directly. Thus administration can be directly intratumoural, suitable especially for solid tumours.

NK cells in general are believed suitable for the methods, uses and compositions of the invention. As per cells used in certain examples herein, the NK cell can be a NK cell obtained from a cancer cell line. Advantageously, a NK cell, preferably treated to reduce its tumourigenicity, for example by rendering it mortal and/or incapable of dividing, can be obtained from a blood cancer cell line and used in methods of the invention to treat blood cancer.

To render a cancer-derived cell more acceptable for therapeutic use, it is generally treated or pre-treated in some way to reduce or remove its propensity to form tumours in the patient. Specific modified NK cell lines, e.g. NK-92 cells, are safe because they can be rendered incapable of division; they are irradiated and retain their killing ability but die within about 3-4 days. Specific cells and cell lines are hence incapable of proliferation, e.g. as a result of irradiation. Treatments of potential NK cells for use in the methods herein include irradiation to prevent them from dividing and forming a tumour *in vivo* and genetic modification to reduce tumourigenicity, e.g. to insert a sequence encoding a suicide gene that can be activated to prevent the cells from dividing and forming a tumour *in vivo.* Suicide genes can be turned on by exogenous, e.g. circulating, agents that then cause cell death in those cells expressing the gene. A further alternative is the use of monoclonal antibodies targeting specific NK cells of the therapy. CD52, for example, is expressed on KHYG-1 cells and binding of monoclonal antibodies to this marker can result in antibody-dependent cell-mediated cytotoxicity (ADCC) and KHYG-1 cell death.

As discussed in an article published by Suck et al, 2006, cancer-derived NK cells and cell lines are easily irradiated using irradiators such as the Gammacell® 3000 Elan. A source of Cesium-137 is used to control the dosing of radiation and a dose-response curve between, for example, 1 Gy and 50 Gy can be used to determine the optimal dose for eliminating the proliferative capacity of the cells, whilst maintaining the benefits of increased cytotoxicity. This is achieved by assaying the cells for cytotoxicity after each dose of radiation has been administered.

There are significant benefits of using an irradiated NK cell line for adoptive cellular immunotherapy over the well-established autologous or MHC-matched T cell approach. Firstly, the use of a NK cell line with a highly proliferative nature means expansion of modified NK cell lines can be achieved more easily and on a commercial level. Irradiation of the modified NK cell line can then be carried out prior to administration of the cells to the patient. These irradiated cells, which retain their useful cytotoxicity, have a limited life span and, unlike modified T cells, will not circulate for long periods of time causing persistent side-effects.

Additionally, the use of allogeneic modified NK cells and NK cell lines means that MHC class I expressing cells in the patient are unable to inhibit NK cytotoxic responses in the same way as they can to autologous NK cytotoxic responses. The use of allogeneic NK cells and cell lines for cancer cell killing benefits from the previously mentioned GVL effect and, unlike for T cells, allogeneic NK cells and cell lines do not stimulate the onset of GVHD, making them a much preferred option for the treatment of cancer via adoptive cellular immunotherapy.

DNA, RNA and amino acid sequences are referred to below, in which:
SEQ ID NO: 1 is an example gRNA for DR5;
SEQ ID NO: 2 is an example gRNA for DR4; and
SEQ ID NO: 3 is a second example gRNA for DR4.

### Examples

The present invention is now described in more and specific details in relation to the production of NK cells, modified to exhibit reduced sensitivity to TRAIL-induced cell death and/or more cytotoxic activity and hence improved ability to cause cancer cell death in humans.

For related examples of knockout / knockdown of checkpoint inhibitory receptor function and knock in of mutant TRAIL we refer to WO 2017/017184.

### Example 1 - Knockout of NK Cell TRAIL Receptors DR4 and DR5

NK Cells are prepared as follows, having death receptor 5 (DR5) and/or death receptor 4 (DR4) function removed.

gRNA constructs targeting TRAIL-R2 (DR5) and TRAIL-R1 (DR4) are designed (e.g.
SEQ ID NO:1: CCCAUCUUGAACAUACCAG (DR5),
SEQ ID NO:2: AACCGGUGCACAGAGGGUGU (DR4) and
SEQ ID NO:3: AUUUACAAGCUGUACAUGGG (DR4))
and prepared to target endogenous genes encoding DR5 and DR4 gene(s) in NK cells. CRISPR/Cas9 genome editing is then used to knock out the DR5 and/or DR4 target genes.

A total of 3 gRNA candidates are selected for the DR5 gene and their cleavage efficacies in RPMI8226 cells determined. A total of 3 gRNA candidates are selected for the DR4 gene and their cleavage efficacies in HL60 cells determined. RPMI8226 cells and HL60 are electroporated with the gRNA:Cas9 ribonucleoprotein (RNP) complex using Maxcyte® GT and subsequently knockout of DR5 and/or is analyzed by flowcytometry. The cleavage activity of the gRNA is also determined using an *in vitro* mismatch detection assay. T7E1 endonuclease recognizes and cleaves non-perfectly matched DNA, allowing the parental DR5 gene / DR4 gene to be compared to the mutated gene following CRISPR/Cas9 transfection and non-homologous end joining (NHEJ).

The gRNA with highest KO efficiency is selected to further experiments to knockout DR5 / DR4 in primary NK cells, NK cell lines or CD34+ progenitors (for subsequent differentiation and expansion to NK cells). Knockout of DR4 / DR5 is determined by flowcytometry based assays.

### Example 2 - Knockdown of TRAIL Receptors DR4 and DR5 in NK Cells

siRNA knockdown of DR4 and/or DR5 in NK-92 cells, KHYG-1 cells and primary NK cells is performed by electroporation. The Nucleofection Kit T can be used, in conjunction with the Amaxa Nucleofector II, from Lonza, as it is appropriate for use with NK cells and can successfully transfect both dividing and non-dividing cells and achieves transfection efficiencies of up to 90%.

The Nucleofector solution is warmed to room temperature (100ul per sample). An aliquot of culture medium containing serum and supplements is also pre-warmed at 37°C in a 50ml tube. 6-well plates are prepared by adding 4ml of culture medium containing serum and supplements. The plates are pre-incubated in a humidified 37°C / 5% CO₂ incubator.

2x10⁶ cells in 100µl Nucleofection solution are mixed gently with 20µM siRNA solution to achieve a final concentration of 2µM. Air bubbles are avoided during mixing. The mixture is transferred into Amaxa certified cuvettes and placed into the Nucleofector cuvette holder.

The program is run and allowed to finish, and the samples in the cuvettes removed immediately. 500µl pre-equilibrated culture medium is then added to each cuvette. The sample in each cuvette is then gently transferred to a corresponding well of the prepared 6-well plate, in order to establish a final volume of 5ml per well.

The cells are then incubated in a humidified 37°C / 5% CO₂ incubator until transfection analysis is performed. Flow cytometry analysis is performed 72 hours after electroporation, in order to measure DR4 and/or DR5 expression levels. This electroporation protocol is found to reliably result in DR4 and DR5 knockdown in KHYG-1 cells, NK-92 cells and primary NK cells.

### Example 3 - DR4-CD28 / DR5-CD28 Fusion Proteins

The immunomodulatory fusion proteins (IFPs) may comprise the extracellular domain of DR4 or DR5, or a portion thereof, and an intracellular signaling domain of CD28, or a portion thereof. The hydrophobic component may be comprised of the transmembrane domain of either DR4 / DR5 or CD28, or portions thereof. In some DR4-CD28 or DR5-CD28 fusion proteins, the hydrophobic component comprises the transmembrane domain of CD28 and the extracellular component further comprises an extracellular portion of CD28, specifically an extracellular cysteine residue adjacent to the hydrophobic component. The extracellular component may comprise all or a portion of the extracellular domain of DR4 or DR5.

The extracellular component may comprise the entire extracellular domain of DR4 / DR5. The DR4-CD28 or DR5-CD28 constructs thus have the capacity to convert what would typically be an inhibitory signal from the binding of either DR4 or DR5 to TRAIL into a positive signal generated by the CD28 intracellular signaling domain. An exemplary nucleic acid molecule encoding a DR4-CD28 fusion protein or a DR5-CD28 fusion protein comprises the following elements (5' to 3'): Extracellular Component (DR4 / DR5)-Multimerization Domain (CD28 Cysteine)-Hydrophobic Component (CD28 transmembrane)-Intracellular Component (CD28 intracellular).

Nucleic acids encoding the constructs can be generated in-house by PCR then directionally TOPO-cloned into the pENTR™/DTOPO® vector (Invitrogen), and transferred into the retroviral vector pMP71-attR using Gateway® technology (Invitrogen). In some cases, the nucleic acid molecules encoding IFPs are codon optimized before cloning into the pMP71-attR retroviral vector.

### Example 4 - Modified TRAIL Receptor Knockin in NK Cells

NK-92 cells, KHYG-1 cells and primary expanded NK cells are transfected with a gene encoding DR5 or DR4 linked to co-stimulatory domain CD28 (as above) or 4-1BB.

A panel of genes encoding immunomodulatory fusion proteins (IFPs) that contain the DR5 or DR4 extracellular binding domain fused to a 4-1BB or CD28 co-stimulatory domain, rather than the natural death domain, is generated. Specifically, a gene of choice can encode an IFP comprising a DR5 backbone containing 4-1BB or CD28 and CD3zeta endodomains and the transmembrane and stalk region of CD8a. The gene construct is delivered as mRNA for transient expression and using SB vectors for establishing stable long term expression.

After transfection (Nucleofection Kit T used as above) of NK cells with the IFPs, the successfully transfected cells are selected based on their IFP functionality. The selected cells are then incubated and passaged as previously described.

The resulting IFPs expressed in NK cells compete with wildtype DR5 for binding of TRAIL but, unlike wildtype DR5, produce activating signals upon binding, leading to a more cytotoxic NK cell phenotype.

### Example 5 - Increased Resistance of Modified NK Cells to TRAIL-induced Killing

Wildtype NK cells are compared to the genetically modified NK cells of Examples 1, 2, 3 and 4.

### Experimental design I:

The sensitivity of each cell type to TRAIL induced cell death is assessed by flowcytometry upon co-culture with RPMI-8226, MM1S, HL60, Panc-1 and BxPC3. Briefly, (1) wildtype NK cells, (2) NK cells expressing high affinity membrane bound TRAIL ligand DR4^{4C9} or DR5^{E195R;D269H}, (3) NK cells with DR4^{KO} or DR5^{KO} and (4) NK cells with DR4^{KO} or DR5^{KO} expressing high affinity membrane bound TRAIL ligand DR4^{4C9} or DR5^{E195R;D269H} are tested against each cancer target cell type.

### Experimental design II:

The sensitivity of each cell type to TRAIL induced cell death is assessed by flowcytometry upon co-culture with RPMI-8226, MM1S, HL60, Panc-1 and BxPC3. Briefly, (1) wildtype NK cells, (2) NK cells expressing high affinity membrane bound TRAIL ligand DR4^{4C9} or DR5^{E195R;D269H}, (3) NK cells with DR4^{KI-IFP} or DR5^{KI-IFP} and (4) NK cells with NK cells with DR4^{KI-IFP} or DR5^{KI-IFP} expressing high affinity membrane bound TRAIL ligand DR4^{4C9} or DR5^{E195R;D269H} are tested against each cancer target cell type.

### Experimental design III:

The sensitivity of each cell type to TRAIL induced cell death is assessed by flowcytometry upon co-culture with RPMI-8226, MM1S, HL60, Panc-1 and BxPC3. Briefly, (1) wildtype NK cells, (2) NK cells expressing high affinity membrane bound TRAIL ligand DR4^{4C9} or DR5^{E195R;D269H}, (3) NK cells with DR4^{KO} or DR5^{KO} , (4) NK cells with DR4^{KI-IFP} or DR5^{KI-IFP} (5) NK cells with NK cells with DR4^{KO} and DR4^{KI-IFP} or DR5^{KO} and DR5^{KI-IFP} expressing high affinity membrane bound TRAIL ligand DR4^{4C9} or DR5^{E195R;D269H} are tested against each cancer target cell type.

The invention thus provides NK cells and cell lines that are resistant to TRAIL-induced cell death, and production thereof, for use in cancer therapy. This approach involves a combination of knocking down/out TRAIL receptors on NK cells to prevent fratricide by neighboring genetically modified NK cells expressing high affinity membrane bound TRAIL ligand. Furthermore, the knockin of TRAIL receptors linked to co-stimulatory domains allows the NK cells to convert the fratricide inducing signals from neighboring NK cells into the activating signal transduction necessary for highly potent NK cell cytotoxicity.

## Claims

1. A natural killer (NK) cell or NK cell line that has been modified to have increased resistance to TRAIL-induced cell death.

2. A NK cell or NK cell line according to claim 1, wherein the increased resistance to TRAIL-induced cell death is by at least 10%, relative to wildtype NK cells.

3. A NK cell or NK cell line according to claim 1 or 2, modified to knockdown or knockout expression of one or more TRAIL receptor genes.

4. A NK cell or NK cell line according to any preceding claim, which expresses or is modified to express a TRAIL receptor linked to a co-stimulatory domain.

5. A NK cell or NK cell line according to claim 3 or 4, wherein the TRAIL receptor is selected from DR4 and DR5.

6. A NK cell or NK cell line according to claim 4 or 5, wherein the co-stimulatory domain is selected from 4-1BB, CD28, DAP-10, DAP-12, CD278 (ICOS) and OX40.

7. A NK cell or NK cell line according to any preceding claim, which further expresses or is modified to express a mutant TRAIL ligand.

8. A NK cell or NK cell line according to claim 7, wherein the mutant TRAIL ligand has an increased affinity for TRAIL receptors, e.g. DR4 and/or DR5.

9. A NK cell or NK cell line according to any preceding claim, wherein the cell line is NK-92 or KHYG-1, or a derivative thereof.

10. A NK cell or NK cell line according to any preceding claim for use in treating cancer in a patient.

11. A NK cell or NK cell line for use according to claim 10, wherein the cancer is a blood cancer.

12. A NK cell or NK cell line for use according to claim 11, wherein the blood cancer is acute lymphocytic leukemia (ALL), acute myeloid leukemia (AML), chronic lymphocytic leukemia (CLL), chronic myeloid leukemia (CML), Hodgkin's lymphoma, non-Hodgkin's lymphoma, including T-cell lymphomas and B-cell lymphomas, asymptomatic myeloma, smoldering multiple myeloma (SMM), active myeloma or light chain myeloma.

13. A NK-92 or KHYG-1 cell modified to express a TRAIL receptor linked to a co-stimulatory domain.

14. A NK-92 or KHYG-1 cell according to claim 13, wherein the TRAIL receptors are selected from DR4 and DR5.

15. A NK-92 or KHYG-1 cell according to claim 13 or 14, wherein the co-stimulatory domain is selected from 4-1BB, CD28, DAP-10, DAP-12, CD278 (ICOS) and OX40.
